# EUROPEAN PATENT APPLICATION

(11) **EP 4 046 639 A1**
(43) Date of publication of application: **24.08.2022**
(21) Application number: 21158504.7
(22) Date of filing: 22.02.2021
(51) Int. Cl.: A61K 31/506, A61K 31/573, A61K 45/06, A61P 11/00, A61P 31/14

(54) **PREVENTION OF PULMONARY VASCULAR LEAK IN COVID-19**

(71) Applicant: Stichting VUmc, 1081 HV Amsterdam (NL)
(72) Inventor: AMAN, Jannes, 1081 HV Amsterdam (NL); BOGAARD, Herman Jan, 1081 HV Amsterdam (NL); DE MAN E/V HANDOKO, Frances Sarah, 1081 HV Amsterdam (NL); DUIJVELAAR, Erik, 1081 HV Amsterdam (NL); VONK NOORDEGRAAF, Antonie, 1081 HV Amsterdam (NL)
(74) Representative: V.O.

(57) **Abstract**

The invention relates to an inhibitor of Abelson Tyrosine-Protein Kinase 2 (*Abl2*), for use in a method of treating an individual suffering from, or at risk of suffering from, virally induced pneumonitis.

## Description

### FIELD:

The invention relates to the field of Covid-19 treatment.

### INTRODUCTION

Hypoxemic respiratory failure is one of the major complications of severe acute respiratory syndrome coronavirus 2 (SARS-CoV2) infection, and is responsible for most of hospitalizations, intubations and mortality in coronavirus disease 2019 (Covid-19) [Wiersenga et al., 2020. JAMA. 324: 782-793]. The clinical picture of lung involvement in Covid-19 is pneumonitis. From the start of the pandemic, radiological investigations revealed extensive ground glass opacities suggestive of diffuse pulmonary edema. Postmortem studies demonstrated that endothelial injury is a key feature of Covid-19 pneumonitis and results from direct infection of pulmonary endothelial cells [Varga et al., 2020. Lancet 395: 1417-1418; Ackermann et al., 2020. N Engl J Med 383: 120-128], disruption of the endothelial barrier and widespread endothelial apoptosis [Ackermann et al., 2020. N Engl J Med 383: 120-128].

Therapeutic strategies that have been evaluated in larger trials for Covid-19 include antiviral treatment and immunomodulatory drugs. Remdesivir shortened the time to recovery in patients hospitalized with Covid-19 in the ACTT-1 trial [Beigel et al., 2020. N Engl J Med 383: 1813-1826], but this finding was not replicated in the WHO Solidarity trial [Pan et al., 2021. N Engl J Med 384: 497-511]. Neither study demonstrated a survival benefit from remdesivir treatment. Likewise, other antiviral intervention, including (hydroxy)chloroquine [Horby et al., 2020. N Engl J Med NEJMoa2021436; Pan et al., 2021. N Engl J Med 384: 497-511], interferon [Pan et al., 2021. N Engl J Med 384: 497-511] and lopinavir-ritonavir [Cao et al., 2020. N Engl J Med 382: 1787-1799; Pan et al., 2021. N Engl J Med 384: 497-511], failed to show benefit in Covid-19. As an alternative to antiviral treatment, and based on the recognition of a hyperinflammatory signature in patients with a worse outcome, other investigators have aimed to modulate the immune response in Covid-19. Dexamethason reduced Covid-19 mortality from 24 to 20% in hospitalized patients and from 40 to 30% in patients in the intensive care unit (ICU) [Pan et al., 2021. N Engl J Med 384: 497-511]. Most recent data from the REMAP-CAP platform indicate that antibody-mediated blockage of Il-6 with tocilizumab or sarilumab reduces mortality in ICU patients [Gordon et al., 2021. MedRxiv doi.org/10.1101/2021.01.07.21249390].

Despite the direct contribution of alveolar edema to hypoxemia and adverse outcomes like intubation or death in Covid-19, treatment strategies attenuating or reversing this type of lung damage have so far not been evaluated. One of the approaches to accomplish this effect, is treatment with the antileukemic drug imatinib [Wiersenga et al., 2020. JAMA 324: 782-793]. The Abl kinase inhibitor imatinib protects against capillary leak and alveolar edema caused by inflammatory stimuli. After a first observation in 2008 of a patient recovering from respiratory failure after imatinib treatment, subsequent *in vitro* and *in vivo* studies revealed that imatinib protects the endothelial barrier under inflammatory conditions [Su et al., 2008. Nat Med 14: 731-7; Aman et al., 2012. Am J Respir Crit Care Med 188: 1171-3; Chislock and Pendergast, 2013. PLoS One 8: e85231; Letsiou et al., 2015. Am J Physiol Lung Cell Mol Physiol 308: L259-269; Langer et al., 2019. J Clin Invest 129: 4691-4707]. Later case reports confirmed the protective effect of imatinib in clinical forms of alveolar edema [Carnevale-Schianca et al., 2011. J Clin Oncol 29: e691-3; Aman et al., 2012. Am J Respir Crit Care Med 188: 1171-3; Langberg et al., 2018. Acta Oncol 57: 1401-1406]. In addition, a single Covid-19 patient has been treated with imatinib [Morales-Ortega et al., 2020. Clin Immunol 218: 108518].

Pulmonary edema in Covid-19 pneumonitis has its specific causes and mechanisms which are not found in other conditions. First, COVID-19 is characterized by a unique form of hyperinflammation that resembles Kawasaki disease in children [Viner and Whittaker, 2020. Lancet 395: 1741-1743]. A further difference is that pulmonary endothelial cells express Angiotensin I Converting Enzyme 2 (ACE2), the receptor for SARS-CoV2 [Zhou et al., 2020. Nature 579: 270-273], so that SARS-CoV2 is able to infect and kill these cells., resulting in direct, SARS-CoV2-mediated damage of the pulmonary vascular endothelium. In addition, it was observed that macrophage-derived cytokines, which are released after stimulation of lung-resident macrophages stimulated by Covid-19 specific antibodies, led to significant disruption of the endothelial layer [Hoepel et al., 2020. BioRxiv 2020.07.13.190140].

In view of the importance of SARS-CoV2-mediated pneumonitis, which may be caused by direct virus-mediated damage of the pulmonary vascular endothelium, there is a clear need for an effective treatment of such virus-induced pneumonitis, which may reduce the duration of mechanical ventilation and the length of stay on an Intensive Care Unit (ICU), and which may help to reduce Covid-19 associated mortalities.

### BRIEF DESCRIPTION OF THE INVENTION

The invention is directed to the surprising finding that oral imatinib reduces the number of adverse events such as duration of mechanical ventilation and duration of ICU stay, and reduces mortality, of patients that were hospitalized with PCR-proven SARS-CoV2 infection and requiring oxygen suppletion.

The invention therefore provides an inhibitor of Abelson Tyrosine-Protein Kinase 2 (*Abl2*), for use in a method of treating an individual suffering from, or at risk of suffering from, virally induced pneumonitis.

Inhibitors of Abl2, such as imatinib, have been reported to protect against capillary leak and alveolar edema caused by inflammatory stimuli (WO2012/150857). It was shown that an Abl2-kinase inhibitor such as imatinib protects against endothelial barrier dysfunction during thrombin stimulation by preserving adherens junction integrity and cell-cell adhesion. This situation differs considerably from virally induced pneumonitis, which is characterized by endothelial injury as a result of direct infection of endothelial cells by the virus inducing said pneumonitis. It can be questioned whether adherens junctions and cell-cell adhesions can be preserved at all when endothelial cells are damaged by viral infection.

As presented in this application, treatment with imatinib did not change the overall time to liberation from supplemental oxygen and mechanical ventilation, the primary endpoints of the study. Imatinib had no effect on the total length of hospital stay. However, secondary endpoint analysis revealed a significant reduction in 28-day mortality rate in a subgroup of patients treated with imatinib group, which paralleled reductions in the duration of mechanical ventilation and the length of stay at an intensive care unit.

In contrast to what would be expected from a compound that is reported to preserve adherens junction integrity and cell-cell adhesion, imatinib did not reduce the time to recovery from respiratory support

The invention further provides an inhibitor of *Abl2,* for use in a method of reducing mortality of an individual suffering from, or at risk of suffering from, virally induced pneumonitis.

The invention further provides an inhibitor of *Abl2,* for use in a method of reducing duration of mechanical ventilation of an individual suffering from, or at risk of suffering from, virally induced pneumonitis.

The invention further provides an inhibitor of *Abl2,* for use in a method of reducing the number of adverse events of an individual suffering from, or at risk of suffering from, virally induced pneumonitis. Said adverse events comprise one or more of hyperglycemia, thrombo-embolic events, decreased lymphocyte count, alkalosis and/or acute respiratory distress syndrome.

In methods of the invention, said pneumonitis is induced by severe acute respiratory syndrome coronavirus 2 (SARS-CoV-2).

In methods of the invention, said inhibitor is an inhibitor of tyrosine kinase activity of the protein encoded by *Abl2,* preferably imatinib.

In methods of the invention, said *Abl2* tyrosine kinase inhibitor is provided for a time period of between 1 day and 4 weeks, preferably for a time period of between 2 days and 2 weeks.

In methods of the invention, said *Abl2* tyrosine kinase inhibitor is combined with a steroid, such as a glucocorticoid. Said steroid preferably is selected from hydrocortisone and dexamethasone.

### LEGENDS TO THE DRAWINGS

Figure 1. Outline of the trial. Screening, Randomization and Treatment Assignment.
Figure 2. Kaplan-Meyer analysis of primary outcome (time to liberation of oxygen), and secondary endpoints (mortality and mechanical ventilation).

### DETAILED DESCRIPTION OF THE INVENTION

### Definitions

The term Abelson Tyrosine-Protein Kinase (Abl)-related gene *Abl2,* as is used herein, refers to a gene on chromosome 1q25.2, which encodes a non-receptor tyrosine-protein kinase that is involved in processes linked to cell growth and survival such as cytoskeleton remodeling in response to extracellular stimuli, cell motility and adhesion and receptor endocytosis. The gene is known under HGNC entry number 77, Entrez Gene entry number 27, and Ensembl entry number ENSG00000143322. The encoded protein Abl2 is known as UniProtKB P4268.

The term method of treating, as is used herein, refers to the application of an agent, such as a drug, in an attempt to cure or mitigate a disease, condition, or injury, in the present case to cure or mitigate virally induced pneumonitis.

The term virally induced pneumonitis, as is used herein, refers to an inflammation of lung tissue that is caused by a viral infection. A virally induced pneumonitis is characterized by damage of the endothelial vascular barrier as a result of direct infection of endothelial cells by the virus inducing said pneumonitis, and/or as a result of activation by the virus of, for example, macrophages.

The term mechanical ventilation, as is used herein, refers to the use of a ventilator or respirator to improve exchange of oxygen (air) between lungs of an individual and the atmosphere. Mechanical ventilation includes noninvasive ventilation, involving a face mask, and invasive ventilation, involving endotracheal intubation. In general, mechanical ventilation is considered when an individual cannot maintain adequate oxygenation. Concerning findings include one or more of a respiratory rate of more than 30/minute, inability to maintain arterial oxygen saturation > 90% with fractional inspired oxygen (FIO2) > 0.60, a body fluid pH < 7.25, and PaCO2 > 50 mm Hg.

The term body fluid, as is used herein, refers to a fluid selected from blood, interstitial fluid, lymph, mucus, pericardial fluid, and pleural fluid. A preferred body fluid is blood, including blood derivatives such as serum and plasma.

The term imatinib, as is used herein, includes reference to the compound 4-[(4-methylpiperazin-1-yl)methyl]-N-[4-methyl-3-[(4-pyridin-3-ylpyrimidin-2-yl)amino]phenyl]benzamide, and a pharmaceutically acceptable salt thereof.

The term PD180970, also referred to as PZ0142, as is used herein, includes reference to the compound 6-(2,6-dichlorophenyl)-2-(4-fluoro-3-methylanilino)-8-methylpyrido[2,3-d]pyrimidin-7-one, and a pharmaceutically acceptable salt thereof.

The term asciminib, as is used herein, includes reference to the compound N-[4-[chloro(difluoro)methoxy] phenyl] -6- [(3R)-3-hydroxypyrrolidin-1-yl]-5-(1H-pyrazol-5-yl)pyridine-3-carboxamide, and a pharmaceutically acceptable salt thereof.

The term dasatinib, as is used herein, includes reference to the compound N-(2-chloro-6-methylphenyl)-2-[[6-[4-(2-hydroxyethyl)piperazin-1-yl]-2-methylpyrimidin-4-yl]amino]-1,3-thiazole-5-carboxamide, and a pharmaceutically acceptable salt thereof.

The term nilotinib, as is used herein, includes reference to the compound 4-methyl-N-[3-(4-methylimidazol-1-yl)-5-(trifluoromethyl)phenyl]-3-[(4-pyridin-3-ylpyrimidin-2-yl)amino]benzamide, and a pharmaceutically acceptable salt thereof.

The term bosutinib, as is used herein, includes reference to the compound 4-(2,4-dichloro-5-methoxyanilino)-6-methoxy-7-[3-(4-methylpiperazin-1-yl)propoxy]quinoline-3-carbonitrile, and a pharmaceutically acceptable salt thereof.

The term ponatinib, as is used herein, includes reference to the compound 3-(2-imidazo[1,2-b]pyridazin-3-ylethynyl)-4-methyl-N-[4-[(4-methylpiperazin-1-yl)methyl]-3-(trifluoromethyl)phenyl]benzamide, and a pharmaceutically acceptable salt thereof.

### Methods of treatment

Provided herein is an inhibitor of Abelson Tyrosine-Protein Kinase 2 (Abl2), for use in a method of treating an individual suffering from, or at risk of suffering from, virally induced pneumonitis. Further provided is an inhibitor of *Abl2* for use in a method of reducing mortality of an individual suffering from, or at risk of suffering from, virally induced pneumonitis. Further provided is an inhibitor of Abl2 for use in a method of reducing duration of mechanical ventilation of an individual suffering from, or at risk of suffering from, virally induced pneumonitis.

The invention further provides methods of treating an individual suffering from, or at risk of suffering from, virally induced pneumonitis, by providing said individual with an effective amount of an inhibitor of Abl2. The invention further provides methods of reducing mortality of an individual suffering from, or at risk of suffering from, virally induced pneumonitis by providing said individual with an effective amount of an inhibitor of Abl2. The invention further provides methods of reducing duration of mechanical ventilation of an individual suffering from, or at risk of suffering from, virally induced pneumonitis by providing said individual with an effective amount of an inhibitor of Abl2.

The observed reduction in mortality may further point to extrapulmonary effects such as, for example, protection of the endothelial barrier in other tissues such as brain, kidney and heart, which will also be infected by the virus.

The invention further provides a use of an inhibitor of Abl2, in the preparation of a medicament for treating an individual suffering from, or at risk of suffering from, virally induced pneumonitis. The invention further provides a use of an inhibitor of Abl2, in the preparation of a medicament for reducing mortality of an individual suffering from, or at risk of suffering from, virally induced pneumonitis. The invention further provides a use of an inhibitor of Abl2, in the preparation of a medicament for reducing duration of mechanical ventilation of an individual suffering from, or at risk of suffering from, virally induced pneumonitis.

Said virally induced pneumonitis is characterized by endothelial injury, especially by endothelial injury as a result of direct infection of endothelial cells by the virus inducing said pneumonitis.

Said virus especially is selected from an influenza virus, Epstein-Barr virus, or a coronavirus such as HCoV-NL63, SARS-CoV, and SARS-CoV-2. Said virus preferably is SARS-CoV-2.

Said inhibitor of Abl2 may be directed to any part of the Abl2 protein that renders the protein inactive. For example, said inhibitor may be targeting the Abl2 myristoyl pocket. An example of such inhibitor is provided by asciminib.

Asciminib preferably is provided at a daily dosage of 10-500 mg, preferably at about 200 mg orally once daily, or 100mg twice daily.

Said inhibitor of Abl2 preferably is an inhibitor of tyrosine kinase activity of Abl2.

Said inhibitor of Abl2 preferably is a specific inhibitor. Said inhibitor preferably exhibits an IC50 concentration for Abl2 kinase activity of less than 1 micromolar, more preferably of 0.5 micromolar or less. Preferably, an inhibitor of Abl2 tyrosine kinase activity specifically inhibits the tyrosine kinase activity of Abl2. Specific inhibition refers to an inhibitor that inhibits Abl2 tyrosine kinase activity at least two times, preferably at least five times, or at least 10 times greater than it inhibits tyrosine kinase activity of a related tyrosine kinase such as Abl1, C-kit and platelet derived growth factor receptor alpha. Preferably, the specific tyrosine kinase activity inhibitor does not significantly inhibit the tyrosine kinase activity of C-kit, PDGFR-alpha and/or C-Abl.

Said Abl2 tyrosine kinase inhibitor preferably is selected from imatinib, PD180970, dasatinib, nilotinib, bosutinib, and ponatinib.

Imatinib preferably is provided at a daily dosage of 100-800 mg, preferably at about 400 mg orally once daily, or 200 mg twice daily. PD180970 preferably is provided at a daily dosage of 10-1000 mg, preferably at about 100-400 mg orally once daily. Dasatinib preferably is provided at a daily dosage of 20-200 mg, preferably at about 100 mg orally once daily. Nilotinib preferably is provided at a daily dosage of 50-1000 mg, preferably at about 600-800 mg orally once daily. Bosutinib preferably is provided at a daily dosage of 100-1000 mg, preferably at about 400-600 mg orally once daily. Ponatinib preferably is provided at a daily dosage of 10-60 mg, preferably at about 45 mg orally once daily. As is known to a person skilled in the art, an individual in need thereof may be provided with an initial dosage of twice the daily recommended dosage on the first day, followed by the recommended dosage as maintenance dose.

Said Abl2 tyrosine kinase inhibitor preferably is selected from imatinib and bosutinib. It has been reported that imatinib and bosutinib induce similar changes in gene expression patterns and molecular pathway alterations of human primary vascular endothelial cells, when compared to dasatinib, ponatinib, and nilotinib (Gover-Proaktor et al., 2019. Leukemia Lymphoma 60: 189-199). In addition, bosutinib has been reported to prevent vascular leakage by reducing focal adhesion turnover and reinforcing junctional integrity (Botros et al., 2020. J Cell Sci 133: jcs240077). A most preferred Abl2 tyrosine kinase inhibitor is or comprises imatinib.

Said Abl2 tyrosine kinase preferably is provided for a time period of between 1 day and 4 weeks, preferably for a time period of between 2 days and 2 weeks, such as 3 days, 4 days, 5 days, 6 days, 7 days, 8 days, 9 days, 10 days, 11 days, 12 days, or 13 days. Said Abl2 tyrosine kinase preferably is daily administered during this time period.

As is presented in the examples, said inhibitor of *Abl2* reduces mortality of an individual suffering from virally induced pneumonitis, with a reduction from 14% in the placebo group (HR 0.51, CI: 95%CI: 0.27-0.95), to 8% in the imatinib group. While the study was not powered to detect differences in mortality, this finding was validated by a reduction in the duration of mechanical ventilation and length of ICU stay.

As is presented in the examples, said inhibitor of *Abl2* reduces duration of mechanical ventilation of an individual suffering from virally induced pneumonitis. In fact, the median duration of mechanical ventilation was 7 days (Interquartile Range (IQR) 3-13 days) in the imatinib group and 12 days (IQR: 6-20 days) in the placebo group (p=0.008).

As is presented in the examples, said inhibitor of *Abl2* reduces the number of adverse events of an individual suffering from virally induced pneumonitis. Said adverse events comprise hyperglycemia, thrombo-embolic events, decreased lymphocyte count, alkalosis and/or acute respiratory distress syndrome.

The beneficial effects of said Abl2 inhibitor are not caused by inhibition of viral infection or viral replication. It has been documented that Abl2 inhibitors such as imatinib and asciminib do not act as anti-SARS-CoV-2 drugs, as imatinib and asciminib had no effect on SARS-CoV-2 infection and replication [Zhao et al., 2020. Leukemia 1038/: s41375-020-01045-9].

In an embodiment, said Abl2 inhibitor is combined with a steroid, preferably a corticosteroid.

It is thought that the provision of a steroid such as a corticosteroid will suppress the immune system, which may further help to control damage of the endothelial barrier.

The invention therefore provides an Abl2 inhibitor for use as a medicament, wherein said inhibitor is combined with a steroid, preferably a corticosteroid.

Said steroid preferably is a glucocorticoid such as hydrocortisone, dexamethasone, prednisolone, prednisone, methylprednisolone, triamcinolone, and/or budesonide. A most preferred steroid is hydrocortisone or dexamethasone.

Said steroid may be administered orally, or by parenteral administration such as by injection, for example intravenous or intramuscular injection, or by infusion.

Hydrocortisone preferably is provided at a daily dosage of 10-500 mg, preferably at about 200 mg once daily, or 100mg twice daily.

Dexamethasone preferably is provided at a daily dosage of 0.25-25 mg, preferably at about 0.75 mg to 9 mg per day. Said daily dosage may be divided over two or more individual dosages, such as three individual dosages or even four individual dosages.

Further provided is a steroid, preferably a corticosteroid, for use as a medicament, wherein said steroid is combined with an Abl2 tyrosine kinase inhibitor.

Further provided is Abl2 inhibitor for use as a medicament, wherein said medicament further comprises a steroid, preferably a corticosteroid.

Further provided is a combination of Abl2 inhibitor and a steroid, preferably a corticosteroid, for use as a medicament.

Further provided is a method of treating an individual suffering from virally induced pneumonitis, comprising providing said individual with an Abl2 inhibitor and a steroid, preferably a corticosteroid.

Further provided is a use of Abl2 inhibitor in the preparation of a medicament for treatment of virally induced pneumonitis, wherein said treatment further comprises a steroid, preferably a corticosteroid.

Further provided is a use of a steroid, preferably a corticosteroid, in the preparation of a medicament for treatment of virally induced pneumonitis, wherein said treatment further comprises an Abl2 inhibitor.

### EXAMPLES

### Example 1

### Materials and methods

### Trial Design

This was a randomized, double-blind and placebo-controlled clinical trial. The trial was conducted between 31st March 2020 and 4th January 2021. The trial was approved by the Medical Ethical Committee of the Amsterdam UMC, location VUMC, and conducted in accordance with Good Clinical Practice and the Declaration of Helsinki. All patients provided written informed consent before randomization. The study was registered at the European Clinical Trial Database (EudraCT Number: 2020-001236-10).

### Patients

Patients were recruited in 13 academic and large non-academic teaching hospitals in The Netherlands. Patients eligible for inclusion were 18 years of age or older, were admitted to the hospital with proven SARS-CoV2 infection (based on a polymerase chain reaction test), and required supplemental oxygen to maintain a peripheral oxygen saturation greater than 94%. Exclusion criteria included, but were not limited to, pre-existing pulmonary disease, pre-existing heart failure (left ventricle ejection fraction <40%), active treatment of a hematological or non-hematological malignancy in the 12 months before inclusion, the presence of cytopenia, and concomitant treatment with medication known to strongly interact with imatinib. Women of childbearing age were asked to undergo pregnancy testing before inclusion.

### Randomization and Intervention

Patients were assigned to placebo or imatinib in a 1:1 ratio. Randomization was performed in the Castor Electronic Data Capturing System (CastorEDC, Castor, Amsterdam, The Netherlands) using blocks of four with stratification per study site. After randomization, patients received oral placebo or imatinib (tablets of 400 mg). After randomization patients received a loading dose of 800 mg (Day 0), followed by 400 mg once daily (Day 1-9). Patients, medical staff and investigators were blinded for the intervention. In case of emergencies, unblinding was performed by an unblinded pharmacist.

### Study procedures

For evaluation of efficacy, recordings were made daily (Day 0 - 9) and on Day 28 of clinical status (discharged, admitted to hospital ward, admitted to ICU, death) and ventilatory/oxygen support (oxygen suppletion, high-flow nasal oxygen, non-invasive ventilation, invasive ventilation, extracorporeal membrane oxygenation). When patients were discharged from hospital before Day 9, clinical status was recorded during telephone contacts on Day 9 and Day 28. Safety evaluation included clinical laboratory testing at baseline and on Days 1, 2, 3, 5, 7 and 9 of blood cell count, electrolytes, kidney function and liver enzymes. An electrocardiogram (ECG) to monitor QT intervals was performed at baseline and on Days 1, 2, 3, 5, 7 and 9. Stopping criteria included: Hemoglobin drop >30% compared to the baseline measurement prior to the loading dose, absolute leukocyte count <2.5 x 109/L, absolute thrombocyte count <100 x 109/L, transaminase elevation of >3x upper limit of normal (ULN) in case of aspartate transaminase (AST) and alanine transaminase (ALT) within reference values at baseline or an elevation of >3x baseline in case of elevated AST/ALT at baseline/inclusion, bilirubine elevation of >1.5x ULN in case of bilirubin levels within reference values at baseline/inclusion or an elevation of >1.5x baseline in case of elevated bilirubin levels at baseline/inclusion, increase in QTc > 100msec compared to baseline. In patients discharged from hospital before Day 9, clinical laboratory testing and ECG measurements were suspended, following guidelines for treatment of chronic myeloid leukemia in an outpatient setting [Hochhaus et al., 2017. Ann Oncol 28: iv41-iv51]. Adverse events and severe adverse events were recorded on a daily basis. All adverse events, grade 3 or higher (as graded by the Common Terminology Criteria for Adverse Events (CTCAE), version 6th November 2017), reported spontaneously by the subject or observed by the investigator or the medical staff were recorded.

### Outcome parameters

The primary outcome was the time to liberation from mechanical ventilation and supplemental oxygen and alive for more than 48h during a 28-day period after randomization. Secondary efficacy parameters included: 28-day mortality, adverse outcome in the 28-day study period (defined as either death or the need for invasive mechanical ventilation during the 28-day study period), and the clinical status at Day 9 and Day 28, according to the 8-point World Health Organization (WHO) ordinal scale for clinical improvement (1 = discharged with full recovery (no home oxygen use); 2 = discharged without full recovery (actual home oxygen use); 3 = hospitalized in a non-ICU hospital ward and not receiving supplemental oxygen; 4 = hospitalized in a non-ICU hospital ward and receiving supplemental oxygen; 5 = hospitalized and receiving noninvasive ventilation or high-flow oxygen; 6 = in the ICU, intubated, and receiving mechanical ventilation; 7 = in the ICU and receiving extracorporeal membrane oxygenation (ECMO) or mechanical ventilation and additional organ support (CVVH); 8 = death). Additional secondary outcome parameters included: the need for ICU admission, the length of ICU admission, the need for invasive ventilation, the length of invasive ventilation, the need for ECMO, the need for and length of non-invasive ventilation, the ratio of peripheral oxygen saturation over the fraction of inspired oxygen (SpO2/FiO2) at Day 1, 2, 3, 4, 5, 7 and 9, as well as the number of days on oxygen supplementation.

Secondary safety parameters included: Incidence of grade 3 adverse and severe adverse events, blood cell count, kidney function, liver enzymes, N-terminal Brain Natriuretic Peptide and corrected QT time (all at Day 0, 1, 2, 3, 5, 7 and 9).

### Data, safety and monitoring

All data were recorded in CastorEDC. Data monitoring was performed by the Clinical Research Bureau, an independent contract research organization of the Amsterdam UMC, location VUMC. The safety of patients was monitored by a Data Safety Monitoring Board (DSMB), which convened after enrolment of 30 patients (April 2020) and 60 patients (May 2020). During these meetings patient safety was assessed based on an unblinded line listing of (severe) adverse events. Subsequently, the DSMB convened after enrolment of 100 patients (October 2020) and 200 patients (December 2020), assessing patient safety and futility based on unblinded line listing of (severe) adverse events and predefined analyses of efficacy endpoints, respectively.

### Data management and Statistical Analysis

Based on an anticipated hazard ratio of 1.39 in the primary outcome in the imatinib group compared to placebo, a one-sided alpha of 0.025 for superiority of the intervention and a beta of 0.20, the sample size was set at 193 patients per arm. All analyses were performed on a modified intention-to-treat (ITT) basis, which included all patients who underwent randomization and received at least one gift of the study medication. All statistical analyses were predefined in the study protocol and the statistical analysis plan.

Baseline demographic variables are expressed as absolute number (proportions) in case of categorical data or as mean and standard deviation or median and interquartile range in case of continuous variables. Imbalances at baseline were carried forward as correction factor in regression analyses of the primary and secondary outcomes. Data were tested for normal distribution using a Kolmogorov-Smirnov test [Massey, 1951. J American Stat Association 46: 68-78].

The primary outcome was analyzed using Kaplan-Mayer curves to plot event rate over time; between group differences were expressed as a hazard ratio with 95% confidence intervals based on Cox regression analyses. Kaplan-Mayer and Cox regression statistics were performed for time-to-event analyses of secondary outcomes.

### Results

### Patients

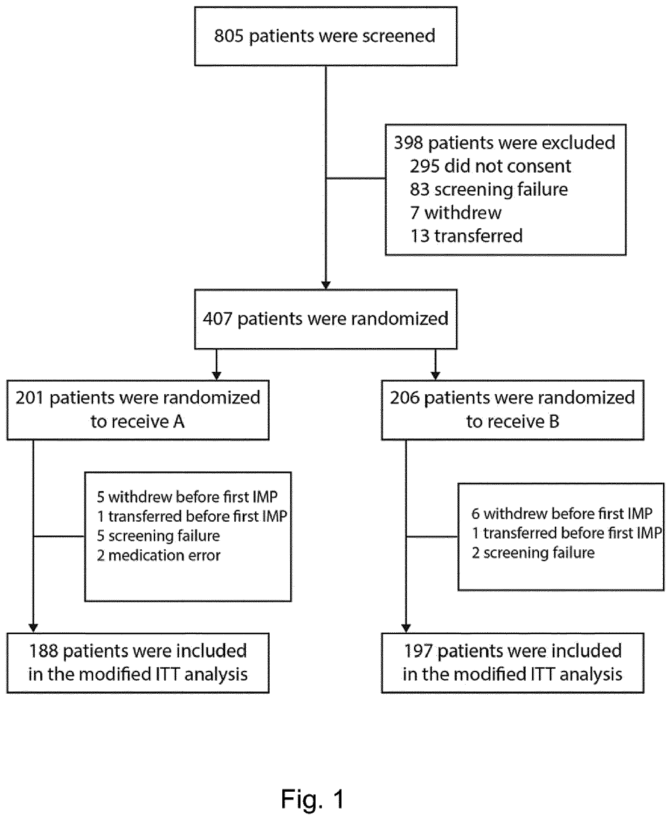

Between 31 March 2020 and 04 Jan 2021 805 patients were screened for eligibility at 13 participating study centers in The Netherlands, and 400 underwent randomization. A total of 11 patients withdrew consent before receiving the first gift of study medication. In addition, after institution of a national system to relocate patients to hospitals with fewer admissions, 2 patients were lost to follow-up after relocation to another hospital not involved in the study. In the final analysis, 188 were randomized to receive placebo and 197 were randomized to receive imatinib (Figure 1).

Baseline characteristics are provided in Table 1. The median age was 64.0 years (interquartile range, 56 to 73 years). The youngest patient was 28 years, the oldest patient in the study population was 93 years. 68.6% of the study population was male, and the median BMI at inclusion was 28.5 kg/m². Common comorbidities included smoking (39.7%), obesity (35.3%), diabetes 24.7%), cardiovascular disease (21.6%) and COPD/asthma (18.4%). Despite randomization, gender, obesity, diabetes and cardiovascular disease were slightly different between the two treatment arms. Clinical presentation at baseline involved chest pain, dyspnea, cough and fever. No differences were observed between the two groups in baseline laboratory measurements, suggesting similar disease severity at presentation.

### Primary outcome

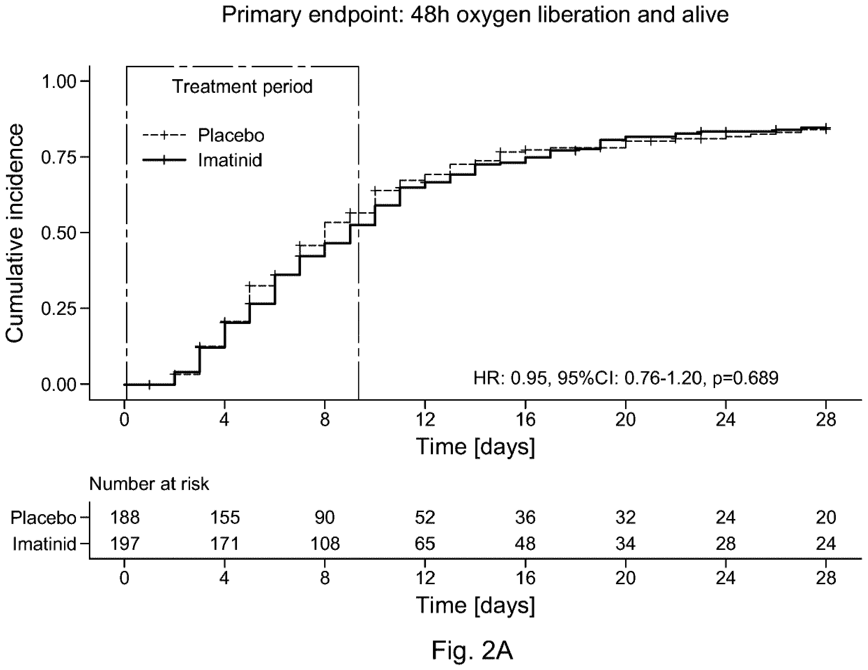

During the 28-day study period, 203 patients (78.7%) became free from oxygen suppletion and mechanical ventilation for >48 hours. The primary outcome was reached in 160 patients receiving imatinib (81%) and in 143 patients receiving placebo (76%). The Kaplan-Meier curve for the primary outcome is shown in Figure 2A. The hazard ratio for reaching the primary outcome was 0.95 in the imatinib group (95% confidence 0.76-1.20, p=0.689). The hazard ratios were also estimated after correction for gender, obesity and diabetes (Table 2).

### Secondary outcomes

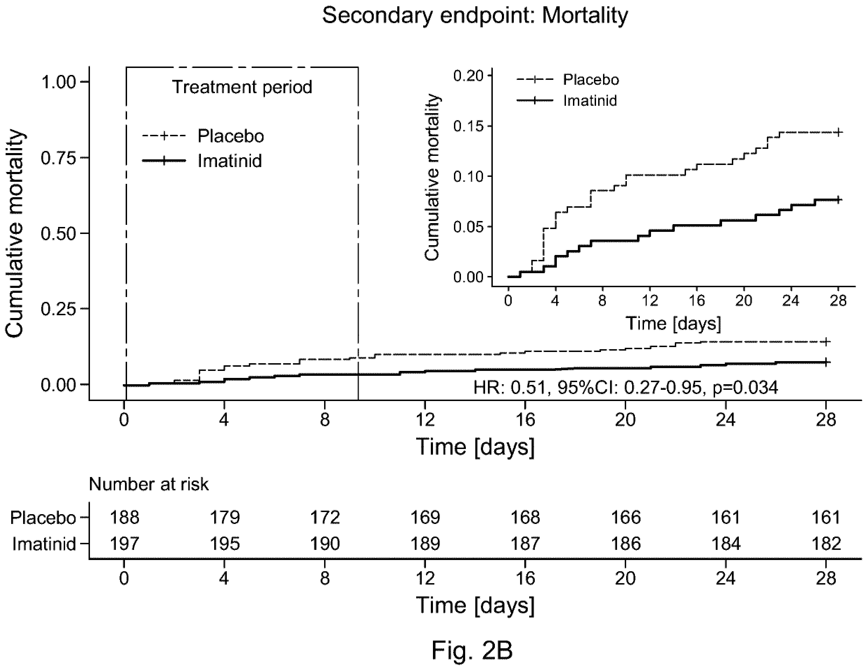

The Kaplan-Meier curves for mortality are provided in Figure 2B. At Day 28, 15 patients (7.6%) had died in the imatinib group, while 27 patients (14.4%) had died in the placebo group. The uncorrected hazard ratio for mortality in the imatinib group was 0.51 (95% CI 0.27-0.95, p=0.034). The hazard ratios for mortality remained significant after correction for baseline imbalances (Table 2).

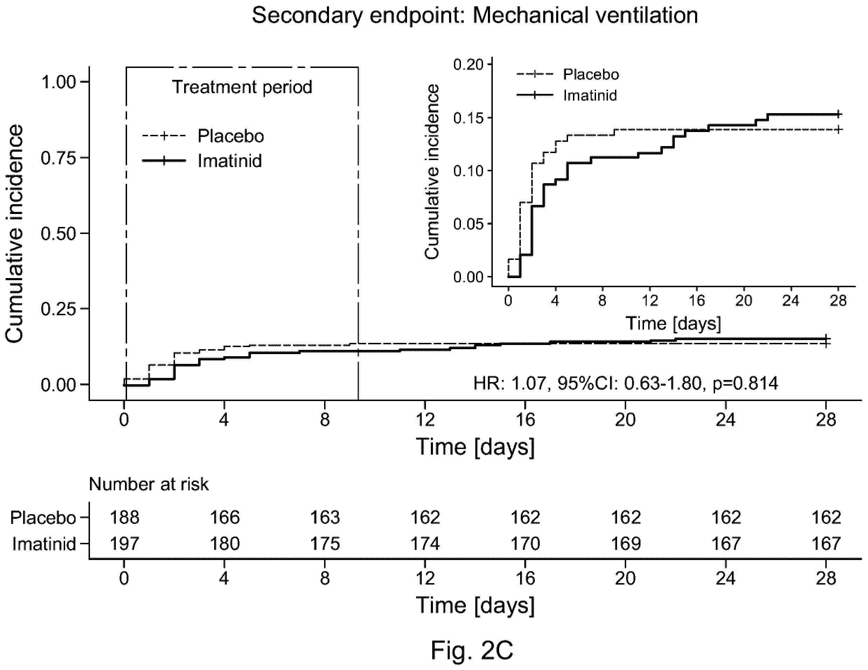

During the 28-day study period, 39 patients (20%) in the imatinib group and 33 patients (18%) in the placebo group were admitted to the Intensive Care Unit (ICU). Of these, 30 patients (15%) in the imatinib group and 26 patients (14%) in the placebo group were intubated and mechanically ventilated (Table 2, Figure 2C). The hazard ratio for mechanical ventilation in the imatinib group was 1.07 (95% CI 0.63-1.80, p=0.814).

The median duration of hospital admission was 7 days (IQR 4-11 days) in the imatinib group and 6 days (IQR: 3-11 days) in the placebo group (p=0.510). The median duration of ICU stay was 8 days (IQR 5-13 days) in the imatinib group and 15 days (IQR: 7-21 days) in the placebo group (p=0.025). The median duration of mechanical ventilation was 7 days (IQR 3-13 days) in the imatinib group and 12 days (IQR: 6-20 days) in the placebo group (p=0.008). Based on the 8-point WHO ordinal scale for clinical recovery at Day 9 and Day 28 of the study period, imatinib use was associated with fewer patients in categories with high disease severity at Day 9 and Day 28 (data not shown).

### Safety evaluation

231 patients (60%) received the full study dose in this study. 77 patients (37.9%) In the imatinib group and 77 patients (41.0%) in the placebo group did not receive the full dose. Frequent reasons for an incomplete study dose included side effects (11.4%), discontinuation due to discharge or transfer to another hospital (9.6%) and discontinuation based on stopping criteria (8.8%). In the imatinib group 30 patients (14.8%) and in the placebo group 14 patients (7.4%) stopped due to side effects. In the imatinib group 11 patients (5.4%) and in the placebo group 23 patients (12.2%) stopped after meeting predefined stop criteria (data not shown).

Safety laboratory measurements and electrocardiograms were performed during the 10-day treatment period. The course of kidney function, liver and cardiac enzymes, as well as QTc times during the first 5 days were recorded (data not shown). Creatinine levels were slightly higher in the imatinib group than in the placebo group after initiation of the study treatment (data not shown). No between group differences were observed for liver enzymes, N-terminal Brain Natriuretic Peptide or QTc time.

During the study grade 3 and 4 adverse events were recorded. In the imatinib group 188 adverse events were reported, in the placebo group 260 adverse events were reported. Most frequent grade 3 adverse events involved thrombo-embolic events, decreased lymphocyte count, alkalosis and hyperglycemia. Most frequent grade 4 adverse events included acute respiratory distress syndrome. No specific adverse events could be related to imatinib treatment. Acute renal failure was observed in 0 patients in the imatinib group versus 2 patients in the placebo group. Hemorrhagic stroke occurred in 1 patient in the imatinib group and in 1 patient in the placebo group (data not shown).

**Table 1. Baseline characteristics and demographics.**

| | **Total** | **Placebo** | **Imatinib** |
|---|---|---|---|
| n | 385 | 188 | 197 |
| Age - median [IQR] | 64 [56-73] | 64 [55-74] | 64 [57-73] |
| BMI- median [IQR] | 28.50 [25.50-32.39] | 29.7 [25.6-32.9] | 27.5 [25.3-31.1] |
| Males - no. % | 264 (68.6) | 118(62.8) | 146 (74.1) |

| ***Comorbidities-no. (%)*** | | | |
|---|---|---|---|
| Current/former smoker | 153 (39.7) | 76 (40.4) | 77 (39.1) |
| BMI>30 | 136 (38.3) | 83 (47,4) | 53 (29,4) |
| Diabetes | 95 (24.7) | 54 (28.7) | 41 (20.8) |
| Cardiovascular disease | 83 (21.6) | 48 (25.5) | 35 (17.8) |
| COPD/Asthma | 71 (18.4) | 33 (17.6) | 38 (19.3) |
| Venous thromboembolism | 10 (2.6) | 5 (2.7) | 5(2.5) |
| Renal failure | 14 (3.6) | 7 (3.7) | 7(3.6) |
| Hepatic disease | 2 (0.5) | 1 (0.5) | 1 (0.5) |
| Rheumatic disease | 29 (7.5) | 18 (9.6) | 11 (5.6) |
| Heart failure | 12 (3.1) | 4 (2.1) | 8 (4.1) |

| ***Medical treatments-no. %*** | | | |
|---|---|---|---|
| Glucose lowering drugs | 94 (24.4) | 54 (28.7) | 40 (20.3) |
| Antihypertensive treatment | 193 (50.1) | 102 (54.3) | 91 (46.2) |
| ACE/ARB | 121 (31.4) | 70 (37.2) | 51 (25.9) |
| Statins | 127 (33.0) | 65 (34.6) | 62 (31.5) |
| Platelet inhibitors | 82 (21.3) | 40 (21.3) | 42 (21.3) |
| Oral anticoagulants | 38 (9.9) | 21 (11.2) | 17 (8.6) |

| ***Clinical presentation-no. %*** | | | |
|---|---|---|---|
| Atypical | 56 (14.5) | 32 (17.0) | 24 (12.2) |
| Chest pain | 61 (15.8) | 27 (14.4) | 34 (17.3) |
| Cough | 292 (75.8) | 141 (75.0) | 151 (76.6) |
| Dyspnea | 318 (82.6) | 158 (84.0) | 160 (81.2) |
| Fever | 245 (63.6) | 112 (59.6) | 133 (67.5) |
| Other flu-like symptoms | 160 (41.6) | 78 (41.5) | 82 (41.6) |
| SpO2/FiO2 | 258 [211-325] | 258 [210-329] | 264 [213-323] |

| ***Laboratory-median [IQR]*** | | | |
|---|---|---|---|
| Hemoglobin - mmol/L | 8.50 [7.80-9.10] | 8.50 [7.80-9.10] | 8.40 [7.80-9.10] |
| C-reactive protein - mg/L | 98.00 [46.95-154.25] | 94.50 [45.35, 149.25] | 102.00 [47.75-157.50] |
| NTproBNP- ng/L | 387.42 [50-385] | 132.50 [50.25-352.00] | 147.00 [49.50-411.50] |
| LDH - U/L | 366.00 [287.00-465.50] | 366.00 [292.50-496.00] | 365.00 [278.75-445.00] |
| Lymphocytes - 10^9/L | 0.90 [0.61-1.20] | 0.94 [0.68-1.28] | 0.90 [0.60-1.19] |
| Neutrophils - 10^9/L | 6.00 [4.20-8.30] | 5.94 [4.40-8.29] | 6.00 [4.10-8.56] |
| Leukocytes - 10^9/L | 7.60 [5.70-10.20] | 7.60 [5.77-10.00] | 7.60 [5.60-10.40] |
| Thrombocytes - 10^9/L | 240.00 [189.00-316.25] | 236.00 [190.00-310.25] | 247.50 [184.00-323.25] |

| ***Medication Initiated at admission* - *no %*** | | | |
|---|---|---|---|
| Dexamethasone | 276 (71.7) | 133 (70.7) | 143 (72.6) |
| Remdesivir | 80 (20.8) | 40 (21.3) | 40 (20.3) |

**Table 2. Time-to-event analysis of primary and secondary outcome parameters.**

| | **HR** | **95%CI** | **p-value** |
|---|---|---|---|
| **Primary endpoint** | | | |
| Imatinib | 0.95 | 0.76-1.20 | 0.689 |
| Imatinib corrected for sex | 1.01 | 0.81-1.28 | 0.900 |
| Imatinib corrected for obesity | 0.99 | 0.78-1.26 | 0.919 |
| Imatinib corrected for diabetes | 0.96 | 0.76-1.20 | 0.690 |
| Imatinib corrected for cardiovascular disease | 0.94 | 0.75-1.18 | 0.591 |

| **Secondary endpoint: Mortality** | | | |
|---|---|---|---|
| Imatinib | 0.51 | 0.27-0.95 | 0.034 |
| Imatinib corrected for sex | 0.47 | 0.25-0.89 | 0.019 |
| Imatinib corrected for obesity | 0.46 | 0.23-0.92 | 0.029 |
| Imatinib corrected for diabetes | 0.54 | 0.29-1.02 | 0.057 |
| Imatinib corrected for cardiovascular disease | 0.54 | 0.29-1.01 | 0.055 |

| **Secondary endpoint: Mechanical ventilation** | | | |
|---|---|---|---|
| Imatinib | 1.07 | 0.63-1.80 | 0.814 |
| Imatinib corrected for sex | 0.98 | 0.58-1.67 | 0.953 |
| Imatinib corrected for obesity | 1.08 | 0.63-1.86 | 0.772 |
| Imatinib corrected for diabetes | 1.13 | 0.66-1.91 | 0.657 |
| Imatinib corrected for cardiovascular disease | 1.06 | 0.62-1.79 | 0.842 |

| | **N** | **Median** | **p-value** |
|---|---|---|---|
| Total days of oxygen supplementation | | | |
| Placebo | 188 (100%) | 5 [3-11] | 0.231 |
| Imatinib | 196 (100%) | 7 [3-12] | |

| Days mechanical ventilation | | | |
|---|---|---|---|
| Placebo | 26 (14%) | 12 [6-20] | 0.008 |
| Imatinib | 30 (15%) | 7 [3-13] | |

| Days at ICU | | | |
|---|---|---|---|
| Placebo | 33 (18%) | 15 [7-21] | 0.025 |
| Imatinib | 39 (20%) | 8 [5-13] | |

## Claims

1. An inhibitor of Abelson Tyrosine-Protein Kinase 2 (Abl2), for use in a method of treating an individual suffering from, or at risk of suffering from, virally induced pneumonitis.

2. An inhibitor of Abl2, for use in a method of reducing mortality of an individual suffering from, or at risk of suffering from, virally induced pneumonitis.

3. An inhibitor of Abl2, for use in a method of reducing duration of mechanical ventilation of an individual suffering from, or at risk of suffering from, virally induced pneumonitis.

4. An inhibitor of Abl2, for use in a method of reducing the number of adverse events of an individual suffering from, or at risk of suffering from, virally induced pneumonitis.

5. The inhibitor for use according to claim 4, wherein said adverse events comprise hyperglycemia, thrombo-embolic events, decreased lymphocyte count, alkalosis and/or acute respiratory distress syndrome.

6. The inhibitor for use according to any one of claims 1-5, wherein said pneumonitis is induced by severe acute respiratory syndrome coronavirus 2 (SARS-CoV-2).

7. The inhibitor for use according to any one of claims 1-6, wherein said inhibitor is an inhibitor of tyrosine kinase activity of the protein encoded by *Abl2.*

8. The inhibitor for use according to any one of claims 1-7, wherein said inhibitor is imatinib.

9. The inhibitor for use according to any one of claims 1-8, wherein said inhibitor is provided for a time period of between 1 day and 4 weeks, preferably for a time period of between 2 days and 2 weeks.

10. The inhibitor for use according to any one of claims 1-9, wherein said inhibitor is combined with a steroid, preferably a glucocorticoid.

11. The inhibitor for use according to claim 10, whereby said steroid is selected from hydrocortisone and dexamethasone.
